# EUROPÄISCHE PATENTSCHRIFT

(11) **EP 0 751 761 B1**
(45) Veröffentlichungstag und Bekanntmachung des Hinweises auf die Patenterteilung: **01.06.2005**
(21) Anmeldenummer: 95914306.6
(22) Anmeldetag: 24.03.1995
(51) Int. Cl.: A61K 7/48

(54) **WIRKSTOFFE FÜR DIE BEHANDLUNG DER SENILEN XEROSIS UND ZUBEREITUNGEN**
ACTIVE SUBSTANCES AND COMPOSITIONS FOR THE THERAPY OF SENILE XEROSIS
SUBSTANCES ACTIVES ET COMPOSITIONS POUR LE TRAITEMENT DU XEROSIS DES PERSONNES AGEES

(30) Priorität: 25.03.1994 DE 4410238
(43) Veröffentlichungstag der Anmeldung: 08.01.1997
(73) Patentinhaber: Beiersdorf Aktiengesellschaft, 20245 Hamburg (DE)
(72) Erfinder: HOPPE, Udo, 22397 Hamburg (DE); SAUERMANN, Gerhard, 24649 Wiemersdorf (DE); SCHREINER, Volker, 20259 Hamburg (DE); STEIGER, Klaus-Michael, CH-8700 Küsnacht (ZH) (CH)
(86) Internationale Anmeldenummer: PCT/EP1995/001118
(87) Internationale Veröffentlichungsnummer: WO 1995/026182

(56) Entgegenhaltungen:
- EP-A- 0 461 333
- WO-A-88/03015
- US-A- 5 378 461
- PATENT ABSTRACTS OF JAPAN vol. 008, no. 011 (C-205) 18. Januar 1984 & JP,A,58 180 410 (SHISEIDO KK) 16. April 1982 in der Anmeldung erwähnt
- PARFUMS, COSMéTIQUES, ARôMES, Bd.46, Nr.74, 1987, PARIS (FRANCE) Seite 6037 G. CERESA 'Emploi de l'Ubiquinone Q10 dans les préparations anti-rides'

## Beschreibung

Die vorliegende Erfindung betrifft insbesondere Wirkstoffe und Zubereitungen zur Behandlung der senilen Xerosis.

Die senile Xerosis wird z.B. durch endogene, genetisch determinierte Faktoren verursacht. In Epidermis und Dermis kommt es alterungsbedingt z.B. zu folgenden Strukturschäden und Funktionsstörungen, die unter den Begriff "Senile Xerosis" fallen:
a) Trockenheit, Rissigkeit, Rauhigkeit und Ausbildung von Trockenheitsfältchen, Juckreiz (Pruritus) und/oder verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung und seniler Xerosis hinausgehen:
b) Sichtbare Gefäßerweiterungen (Couperosis);
c) Schlaffheit und Ausbildung von Falten;
d) lokale Hyper-, Hypo- und Fehlpigmentierungen (z.B. Altersflecken) und
e) vergrößerte Anfälligkeit gegenüber mechanischem Stress (z.B. Rissigkeit).

Die vorliegende Erfindung betrifft insbesondere Wirkstoffe und Produkte zur Behandlung der Folgeschäden der Hautalterung und senilen Xerosis, insbesondere der unter a) bis e) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch W-Strahlen und der Prävention von Hautalterung. Bei 20 bis 40-jährigen wird die Hautrauhigkeit gebessert, indem der Haut Feuchtigkeit gegeben wird.

WO-A-88 03015 offenbart kosmetische Zubereitungen enthaltend Derivate der Ubidecarenone und deren Verwendung gegen Hautalterung.

Aufgabe der vorliegenden Erfindung war es somit, Wege zu finden, die die Nachteile des Standes der Technik vermeiden. Insbesondere soll die Wirkung der Behebung der mit der senilen Xerosis und exogenen Hautalterung verbundenen Schäden dauerhaft, nachhaltig und ohne das Risiko von Nebenwirkungen sein.

Diese Aufgaben werden durch die Erfindung gelöst.

Gegenstand der Erfindung sind topische Zubereitungen mit einem Gehalt an einer Wirkstoffkombination aus i) einer Verbindung oder mehreren Verbindungen aus der Gruppe der Ubichinone und ii) Kreatin.

Die erfindungsgemäßen topischen Zubereitungen können kosmetische oder dermatologische Zubereitungen sein.

Gegenstand der Erfindung ist auch die Verwendung von Wirkstoffkombinationen aus i) einer Verbindung oder mehrerer Verbindungen aus der Gruppe der Ubichinone und ii) Kreatin zur Behandlung der senilen Xerosis und/oder exogen bedingten Hautalterung.

Es hat sich überraschenderweise gezeigt, daß Ubichinone und deren Derivate nicht nur die Haut vor Schäden durch chronologische Hautalterung, insbesondere aber Lichtalterung schützen, sondern auch die Reparatur von bereits entstandenen Schäden der Haut bei seniler Xerosis und exogen bedingter Hautalterung herbeiführen, was in signifikanter Weise Nachteilen des Standes der Technik abhilft. Diese Wirkung dieser Stoffgruppe auf strukturelle Veränderungen der Altershaut ist besonders vorteilhaft.

Mit "Ubichinone" sind hier auch "Ubichinone und deren Derivate" gemeint.

Bei der senilen Xerosis kommt es alterungsbedingt insbesondere zu folgenden Strukturschäden und Funktionsstörungen:
a) Trockenheit, Rauhigkeit und Ausbildung von Hautfältchen, Juckreiz, verminderte Rückfettung durch Talgdrüsen (z.B. nach dem Waschen).
   Bei der exogen bedingten Hautalterung, die z.B. durch UV-Licht und chemische Noxen bewirkt wird, kommt es insbesondere zu den folgenden Störungen:
b) Sichtbare Gefäßerweiterungen (Couperosis);
c) Schlaffheit der Haut und Ausbildung von Falten;
d) lokale Hyper-, Hypo- und Fehlpigmentierungen (z.B. Altersflecken) und
e) vergrößerte Anfälligkeit gegenüber mechanischem Stress (z.B. Rissigkeit).

Die Ubichinone sind aus der Literatur bekannt (z.B. "Römpp Chemie Lexikon", Georg Thieme Verlag Stuttgart, New York, 9. Auflage, S. 4784-4785 oder "The Merck Index", 11th Edition, Merck & Co., Inc. Rahway, N.Y., USA, Abstr. 9751 (1989). Sie werden auch als Mitochinone oder Coenzyme Q bezeichnet. Die Anzahl der Isopren-Einheiten in der Seitenkette wird mit n in der Bezeichnung Coenzyme Q-n angegeben, worin n eine ganze Zahl bedeutet. Bevorzugt werden Ubichinone oder Coenzyme Q-n mit n=0-12, besonders bevorzugt n=1-12 und insbesondere n=6 bis 10. Gegenstand der Erfindung ist also auch der Chinongrundkörper des Ubichinons ohne Isoprensubstituenten. Erfindungsgemäße Ubichinone oder deren Derivate sind z.B. auch Alkyl-Ubichinone, insbesondere 6-Alkyl-Ubichinone mit vorzugsweise C₁-C₁₂-Alkyl-Resten. Bevorzugt wird Decyl-Ubichinon, insbesondere 6-Decyl-Ubichinon oder 2,3-Dimethoxy-5-methyl-6-decyl-1,4-benzochinon.

Ubichinone fungieren bei der biologischen, mitochondralen Oxidation als Elektronenüberträger und spielen so eine bedeutende Rolle beim Energiestoffwechsel der tierischen Zellen. In kosmetischen Zubereitungen finden Ubichinone seit langem Anwendung als Antioxidantien zum Schutz oxidationempfindlicher Substanzen.

Besonders bevorzugt werden die folgenden erfindungsgemäßen Wirkstoffe und Kombinationen damit:
Coenzym Q - 10, Coenzym Q - 9, Coenzym Q - 8, Coenzym Q - 7, Coenzym Q - 6

Die erfindungsgemäßen Wirkstoffe können in den topischen Zubereitungen in Mengen von 0,001 bis 99 Gew.-%, z.B. auch in Mengen von 0,001 bis 50 Gew.-% vorliegen, jeweils bezogen auf das Gesamtgewicht der Zubereitungen.

Vorzugsweise können die erfindungsgemäßen Wirkstoffe in den topischen Zubereitungen in Mengen von 0,01 bis 10 Gew.-%, insbesondere in Mengen von 0,1 bis 1 Gew.-% vorliegen, jeweils bezogen auf das Gesamtgewicht der Zubereitungen.

Ganz besonders bevorzugt enthalten die Hautpflegeprodukte oder Dermatika 0,2 bis 0,4 Gew.-%, insbesondere 0,3 Gew.-% Coenzym Q-10.

Im Rahmen der Anmeldung sind stets Gewichtsprozente bezogen auf 100% Gesamtzusammensetzung des jeweiligen erfindungsgemäßen Hautpflegepräparates oder Dermatikums gemeint.

Erfindungsgemäße topische Zubereitungen oder Zusammensetzungen mit den erfindungsgemäßen Kombinationen und Wirkstoffen sind alle gängigen Anwendungsformen, z.B. Cremes (W/O, O/W, W/O/W), Gele, Lotionen, Milchen.

Die erfindungsgemäßen topischen Zubereitungen können als flüssige, pastöse oder feste Zubereitungen formuliert werden, beispielsweise als wäßrige oder alkoholische Lösungen, wäßrige Suspensionen, Emulsionen, Salben, Cremes, Öle, Pulver oder Stifte. In Abhängigkeit von der gewünschten Formulierung können die Wirkstoffe in pharmazeutische und kosmetische Grundlagen für topische Applikationen eingearbeitet werden, die als weitere Komponenten beispielsweise Ölkomponenten, Fett und Wachse, Emulgatoren, anionische, kationische, ampholyitsche, zwitterionische und/oder nichtionogene Tenside, niedere ein- und mehrwertige Alkohole, Wasser, Konservierungsmittel, Puffersubstanzen, Verdickungsmittel, Duftstoffe, Farbstoffe und Trübungsmittel enthalten. Vorteilhaft können die erfindungsgemäßen Wirkstoffe auch in transdermalen therapeutischen Systemen, insbesondere kubischen Systemen verwendet werden.

Es ist ferner von Vorteil, den Zubereitungen Antioxidantien (z.B. alpha-Tocopherol, Vitamin E und C, Imidazole, alpha-Hydroxycarbonsäuren (z.B. Äpfelsäure, Glycolsäure, Gluconsäure, Salicylsäure sowie deren Derivate) und/oder Eisenkomplexbildner (z.B. EDTA, alpha-Hydroxyfettsäuren) und/oder bekannte UV-Lichtschutzfilter in Mengen von z.B. 0,1 bis 10 Gewichtsprozenten zuzusetzen, um die Stabilität_der oxidationsempfindlichen Ubichinone oder Plastochinone zu gewährleisten.

Auch ist es vorteilhaft, den Zubereitungen insbesondere 0,01 - 10 Gewichtsprozente an Stoffen bzw. Stoffkombinationen des aeroben zellulären Energiestoffwechsels (z.B. zelluläre Energieüberträger (wie Kreatin, Guanin, Guanosin, Adenin, Adenosin, Nicotin, Nicotinamid, Riboflavin), Coenzyme (z.B. Pantothensäure, Panthenol, Liponsäure, Niacin), Hilfsfaktoren (z.B. L-Carnitin, Uridin), Substrate (z.B. Hexosen, Pentosen, Fettsäuren) und intermediäre Stoffwechselprodukte (z.B. Zitronensäure, Pyruvat) und/oder Glutathion zuzusetzen.

Vorteilhaft können erfindungsgemäße Zubereitungen außerdem Substanzen enthalten, die UV-Strahlung im UVA- und/oder im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen. In den Zubereitungen wirken die-UV-Absorber gegenüber den Wirkstoffen als Antioxidantien.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
3-Benzylidencampher-Derivate, vorzugsweise 3-(4-Methylbenzyliden)campher, 3-Benzylidencampher.

### Vorteilhaft wasserlösliche UVB-Filter sind z.B.:

Salze der 2-Phenylbenzimidazol-5-sulfonsäure wie ihr Natrium-, Kalium- oder ihr Triethanolammonium-Salz, sowie die Suffonsäure selbst.

Es kann auch von Vorteil sein, erfindungsgemäße Wirkstoffkombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'tert.Butylphenyl)-3-(4'-methoxyphenyl)propan-1,3-dion und um 1-Phenyl-3-(4'isopropylphenyl)propan-1,3dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung sind also auch die Kombinationen der erfindungsgemäßen Wirkstoffe, insbesondere in den topischen Zubereitungen, mit Antioxydantien, Stoffen des aeroben zellulären Energiestoffwechsels und/oder UV-Absorbern, durch die sich z.B. die Stabilität und die Wirkung der Zubereitung verbessern läßt.

Die vorstehend aufgeführten Beispiele für kombinierbare Wirkstoffe aus den angegebenen Wirkstoffgruppen dienen dazu, die Erfindung zu beschreiben, ohne daß beabsichtigt ist, die Erfindung auf diese Beispiele zu beschränken.

Darüber hinaus können schützende Formulierungsformen angewendet werden, wobei die erfindungsgemäßen Stoffe z.B. in Liposomen, Micellen, Nanosphären usw. aus z.B. hydrierten Amphiphilen, wie z.B. Ceramiden, Fettsäuren, Sphingomyelin und Phosphoglyceriden bzw. in Zyklodextrane eingeschossen (verkapselt) werden. Weiterer Schutz kann durch die Verwendung von Schutzgas (z.B. N₂, CO₂) bei der Formulierung und die Verwendung gasdichter Verpackungsformen erreicht werden.

Weitere Hilfs- und Zusatzstoffe können wasserbindende Stoffe, Verdicker, Füllstoffe, Parfüm, Farbstoffe, Emulgatoren, Wirkstoffe wie Vitamine, Konservierungsmittel, Wasser und/oder Salze sein.

Bei der Verarbeitung der Ubichinone und anderer oxidationsempfindlicher Stoffe sollte die Temperatur nicht über 40°C liegen. Ansonsten sind die üblichen Maßregeln zu beachten, die dem Fachmann bekannt sind.

Die erfindungsgemäßen Stoffgruppen lassen sich so in alle kosmetischen Grundlagen einarbeiten. Grundsätzlich sind allerdings W/O- und O/W-und W/O/W-Emulsionen bevorzugt. Besonders vorteilhaft können erfindungsgemäße Kombinationen in Pflegeprodukte wie beispielsweise O/W-Cremes, W/O-Cremes, O/W-Lotionen, W/O-Lotionen usw. eingesetzt werden.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die folgenden Beispiele sind nicht erfindungsgemäß und dienen dazu, die Erfindung zu beschreiben.

Die angegebenen Teile sind Gewichtsteile.

### Beispiel I

| Hautcreme vom W/O-Typ | |
|---|---|
| | Gew.-Teile |
| Vaseline DAB 9 | 13 |
| Glycerin DAB 9 | 6,3 |
| Wasser (VES, vollentsalzt) | 34,4 |
| Paraffinöl (Mineralöl 5E,Shell) | 35 |
| Cetearylalkohol/PEG-40-Castor Oil/Natriumcetearylsulfat (Emulgade F, Henkel KGaA) | 2,5 |

Zu der 75°C warmen Fettphase wird die 75°C warme Wasserphase gegeben, verrührt und homogenisiert bis eine gleichmäßig weiße Creme entstanden ist. 0,3 Teile Coenzym Q10 werden in 8,5 Teilen Paraffinöl gelöst und zu der auf etwa 40°C abgekühlten Creme gegeben und verrührt, bis eine gleichmäßige hellgelbe Creme entstanden ist.

Beispiel I hat folgende endgültige Zusammensetzung:

| | Gew.-Teile |
|---|---|
| Vaseline DAB 9 | 13 |
| Glycerin DAB 9 | 6,3 |
| Wasser VES | 34,4 |
| Paraffinöl (Mineralöl 5E,Shell) | 43,5 |
| Cetearylalkohol/PEG-40-Castor Oil/Natriumcetearylsulfat (Emulgade F, Henkel KGaA) | 2,5 |
| Coenzym Q₁₀ | 0,3 |
| | 100 |

### Beispiel II

| Hautcreme vom W/O-Typ | |
|---|---|
| | Gew.-Teile |
| PEG-1 Glyceryl-Oleostearat + Paraffinwachs | 8 |
| Vaseline DAB | 2,8 |
| Paraffinöl (Mineralöl 5E,Shell) | 9,9 |
| Paraffinwachs/Paraffin | 1,8 |
| Ceresin | 2,2 |
| Octyldodecanol (Eutanol G) | 10 |
| Propylenglycol | 1 |
| Glycerin | 1 |
| Magnesiumsulfat | 0,7 |
| Wasser VES | 59,7 |
| Summe Additive (Parfüm, Konservierung, Stabilisation) | 0,8 |

Zu der 75°C warmen Fettphase wird die 75°C warme Wasserphase gegeben, verrührt und homogenisiert bis eine gleichmäßig weiße Creme entstanden ist. 0,36 Teile Coenzym Q10 und 0,04 Teile Coenzym Q6 werden in 2 Teilen Paraffinöl gelöst und zu der auf etwa 40°C abgekühlten Creme gegeben und verrührt, bis eine gleichmäßige hellgelbe Creme entstanden ist.

Beispiel II hat folgende endgültige Zusammensetzung:

| | Gew.-Teile |
|---|---|
| PEG-1 Glyceryl-Oleostearat + Paraffinwachs | 8 |
| Vaseline DAB | 2,8 |
| Paraffinwachs/Paraffin | 1,8 |
| Paraffinöl (Mineralöl 5E,Shell) | 11,9 |
| Ceresin | 2,2 |
| Octyldodecanol | 10 |
| Coenzym Q₆ | 0,04 |
| Coenzym Q10 | 0,36 |
| Propylenglycol | 1 |
| Glycerin | 1 |
| Magnesiumsulfat | 0,7 |
| Wasser VES | 59,4 |
| Summe Additive (Parfüm, Konservierung, Stabilisation) | 0,8 |
| | 100 |

### Beispiel III

| Hautcreme vom O/W-Typ | |
|---|---|
| | Gew.-Teile |
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |
| Paraffinöl (Mineralöl 5E, Shell) | 5,8 |

Zu der 75°C warmen Fettphase wird die 75°C warme Wasserphase gegeben, verrührt und homogenisiert bis eine gleichmäßig weiße Creme entstanden ist. 0,54 Teile Coenzym Q10 und 0,36 Teile Coenzym Q6 werden in 2 Teilen Paraffinöl gelöst und zu der auf etwa 40°C abgekühlten Creme gegeben und verrührt, bis eine gleichmäßige hellgelbe Creme entstanden ist.

Beispiel III hat folgende endgültige Zusammensetzung:

| | Gew.-Teile |
|---|---|
| Octyldodecanol (Eutanol G, Henkel KGaA) | 9,3 |
| Cetearylalkohol/PEG-40-Castor Oil/Natriumcetearylsulfat (Emulgade F, Henkel KGaA) | 3,7 |
| Wasser VES | 73,7 |
| Glycerin DAB 9 | 4,6 |
| Paraffinöl (Mineralöl eE, Shell) | 7,8 |
| Coenzym Q6 | 0,36 |
| Coenzym Q₁₀ | 0,54 |
| | 100 |

### Beispiel IV

| O/W-Lotion | |
|---|---|
| | Gew.-Teile |
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Mineralöl 5E, Shell) | 10,1 |
| Propylenglycol | 1 |
| Glycerin | 1 |
| Wasser VES | 74,3 |
| Summe Additive (Parfüm, Konservierung, Stabilisation) | 0,8 |

Zu der 75°C warmen Fettphase wird die 75°C warme Wasserphase gegeben, verrührt und homogenisiert bis eine gleichmäßig weiße Lotion entstanden ist. 0,2 Teile Coenzym Q10 und 0,2 Teile Coenzym Q6 werden in 4 Teilen Paraffinöl gelöst und zu der auf etwa 40°C abgekühlten Lotion gegeben und verrührt, bis eine gleichmäßige hellgelbe Lotion entstanden ist.

Beispiel IV hat folgende endgültige Zusammensetzung:

| | Gew.-Teile |
|---|---|
| Steareth-2 | 3 |
| Steareth-21 | 2 |
| Cetearylalkohol/PEG-40-Castor Oil/Natriumcetearylsulfat (Emulgade F, Henkel KGaA) | 2,5 |
| Paraffinöl (Minesalöl 5E, Shell) | 14,1 |
| Propylenglycol | 1 |
| Coenzym Q6 | 0,2 |
| Coenzym Q₁₀ | 0,2 |
| Glycerin | 1 |
| Wasser VES | 74,3 |
| Summe Additive (Parfüm, Konservierung, Stabilisation) | 0,8 |
| | 100 |

### Beispiel V

| O/W-Lotion | |
|---|---|
| | Gew.-Teile |
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl 5E, Shell) | 5,7 |

Zu der 75°C warmen Fettphase wird die 75°C warme Wasserphase gegeben, verrührt und homogenisiert bis eine gleichmäßig weiße Creme entstanden ist. 0,04 Teile Coenzym Q10 und 0,36 Teile Coenzym Q6 werden in 5 Teilen Paraffinöl gelöst und zu der auf etwa 40°C abgekühlten Creme gegeben und verrührt bis eine gleichmäßige hellgelbe Creme entstanden ist.

Beispiel V hat folgende endgültige Zusammensetzung:

| | Gew.-Teile |
|---|---|
| Octyldodecanol (Eutanol G, Henkel KGaA) | 5,6 |
| Cetearylalkohol/PEG-40-Castor Oil/Natriumcetearylsulfat (Emulgade F, Henkel KGaA) | 8,9 |
| Cetearylisononanoat (Cetiol 5N, Henkel KGaA) | 7,5 |
| Wasser VES | 62,3 |
| Glycerin DAB 9 | 4,7 |
| Paraffinöl (Mineralöl 5E, Shell) | 10,7 |
| Coenzym Q6 | 0,04 |
| Coenzym Q₁₀ | 0.36 |
| | 100 |

### Beispiel VI

| Hautöl | |
|---|---|
| | Gew.-Teile |
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA) | 20 |
| Paraffinöl (Mineralöl 5E, Shell) | 35 |
| Coenzym Q₆ | 2 |
| | 100 |

Die Komponenten werden bei 25°C verrührt, bis eine gleichmäßige, klare Mischung entstanden ist

### Beispiel VII

| Hautöl | |
|---|---|
| | Gew.-Teile |
| Glyceryltricaprylat (Miglyol 812, Dynamit Nobel) | 21 |
| Hexyllaurat (Cetiol A, Henkel KGaA) | 20 |
| Octylstearat (Cetiol 886, Henkel KGaA) | 20 |
| Paraffinöl (Mineralöl 5E, Shell) | 35 |
| Coenzym Q₆ | 2 |
| | 100 |

Die Herstellung erfolgt wie in Beispiel VI angegeben.

## Patentansprüche

1. Zubereitungen mit einem Gehalt an einer Wirkstoffkombination aus
i) einer Verbindung oder mehreren Verbindungen aus der Gruppe der Ubichinone und
ii) Kreatin.

2. Zubereitungen gemäß einem der vorgehenden Ansprüche, **dadurch gekennzeichnet, daß** die Ubichinone 0 bis 12 Isopreneinheiten und/oder gegebenenfalls Alkyl-Reste besitzen.

3. Zubereitungen gemäß einem der vorgehenden Ansprüche, **dadurch gekennzeichnet, daß** die Ubichinone 9 oder 10 Isopreneinheiten besitzen.

4. Zubereitungen gemäß einem der vorgehenden Ansprüche, **dadurch gekennzeichnet, daß** sie Antioxidantien, Stoffe des aeroben zellulären Energiestoffwechsels und/oder UV-Absorber enthalten.

5. Zubereitungen gemäß einem der vorgehenden Ansprüche, **dadurch gekennzeichnet, daß** sie kosmetische oder dermatologische Zubereitungen, insbesondere W/O-, O/W- oder W/O/W-Emulsionen sind.

6. Kosmetische Verwendung topischer Zubereitungen gemäß einem der vorgehenden Ansprüche zur Behandlung der senilen Xerosis und/oder der exogen bedingten Hautalterung.

7. Kosmetische Verwendung topischer Zubereitungen gemäß einem der vorgehenden Ansprüche zur Behandlung einer oder mehrerer der folgenden Erscheinungsbilder der exogen bedingten Hautalterung:
a) Trockenheit, Rissigkeit, Rauhigkeit und Ausbildung von Trockenheitsfältchen, Juckreiz (Pruritus) und/oder verminderte Rückfettung durch Talgdrüsen (z.B. nach Waschen).
b) Sichtbare Gefäßerweiterungen (Couperosis);
c) Schlaffheit und Ausbildung von Falten;
d) lokale Hyper-, Hypo- und Fehlpigmentierungen (z.B. Altersflecken) und
e) vergrößerte Anfälligkeit gegenüber mechanischem Stress (z.B. Rissigkeit).

8. Verwendung von Wirkstoffkombinationen aus
i) einer Verbindung oder mehreren Verbindungen aus der Gruppe der Ubichinone und
ii) Kreatin.
zur Herstellung topischer therapeutischer Zubereitungen zur Behandlung der senilen Xerosis und/oder der exogen bedingten Hautalterung.

9. Verwendung von Wirkstoffkombinationen aus
i) einer Verbindung oder mehreren Verbindungen aus der Gruppe der Ubichinone und
ii) Kreatin.
zur Herstellung topischer therapeutischer Zubereitungen zur Behandlung einer oder mehrerer der folgenden Erscheinungsbilder der exogen bedingten Hautalterung:
a) Trockenheit, Rissigkeit, Rauhigkeit und Ausbildung von Trockenheitsfältchen, Juckreiz (Pruritus) und/oder verminderte Rückfettung durch Talgdrüsen (z.B. nach Waschen).
b) Sichtbare Gefäßerweiterungen (Couperosis);
c) Schlaffheit und Ausbildung von Falten;
d) lokale Hyper-, Hypo- und Fehlpigmentierungen (z.B. Altersflecken) und
e) vergrößerte Anfälligkeit gegenüber mechanischem Stress (z.B. Rissigkeit).

## Claims

1. Formulations having a content of an active compound combination of i) one or more compounds from the group consisting of ubiquinones and ii) creatine.

2. Formulations according to one of the preceding claims, **characterized in that** the ubiquinones have 0 to 12 isoprene units and/or, if appropriate, alkyl radicals.

3. Formulations according to Claim 1, **characterized in that** the ubiquinones have 9 or 10 isoprene units.

4. Formulations according to one of the preceding claims, **characterized in that** they comprise antioxidants, substances of aerobic cell energy metabolism and/or UV absorbers.

5. Formulations according to one of the preceding claims, **characterized in that** they are cosmetic or dermatological formulations, in particular W/O, O/W or W/O/W emulsions.

6. Cosmetic use of topical formulations according to one of the preceding claims for treatment of senile xerosis and/or exogenous ageing of the skin.

7. Cosmetic use of topical formulations according to one of the preceding claims for treatment of one or more of the following phenomena of exogenous ageing of the skin:
a) dryness, cracking, roughness and development of dryness wrinkles, itching (pruritus) and/or reduced re-oiling by sebaceous glands (for example after washing).
b) visible dilation of vessels (couperosis);
c) flaccidity and development of wrinkles;
d) local hyper- and hypopigmentations and faulty pigmentations (for example senile keratosis) and
e) increased susceptibility to mechanical stress (for example cracking).

8. Use of active compound combinations of
i) one or more compounds from the group consisting of ubiquinones and
ii) creatine
for the preparation of topical therapeutic formulations for treatment of senile xerosis and/or exogenous ageing of the skin.

9. Use of active compound combinations of
i) one or more compounds from the group consisting of ubiquinones and
ii) creatine
for the preparation of topical therapeutic formulations for treatment of one or more of the following phenomena of exogenous ageing of the skin:
a) dryness, cracking, roughness and development of dryness wrinkles, itching (pruritus) and/or reduced re-oiling by sebaceous glands (for example after washing).
b) visible dilation of vessels (couperosis);
c) flaccidity and development of wrinkles;
d) local hyper- and hypopigmentations and faulty pigmentations (for example senile keratosis) and
e) increased susceptibility to mechanical stress (for example cracking).

## Revendications

1. Préparations ayant une teneur en une association de substances actives constituée
i) d'un composé ou de plusieurs composés choisi(s) dans le groupe des ubiquinones et
ii) de créatine.

2. Préparations selon la revendication 1, **caractérisées en ce que** les ubiquinones comportent de 0 à 12 unités isoprène et/ou éventuellement des radicaux alkyle.

3. Préparations selon l'une quelconque des revendications précédentes, **caractérisées en ce que** les ubiquinones comportent 9 ou 10 unités isoprène.

4. Préparations selon l'une quelconque des revendications précédentes, **caractérisées en ce qu'**elles contiennent des antioxydants, des substances du métabolisme énergétique cellulaire aérobie et/ou des absorbeurs d'UV.

5. Préparations selon l'une quelconque des revendications précédentes, **caractérisées en ce qu'**elles sont des préparations cosmétiques ou dermatologiques, en particulier des émulsions E/H, H/E ou E/H/E.

6. Utilisation cosmétique de préparations topiques selon l'une quelconque des revendications précédentes, pour le traitement de la xérose sénile et/ou du vieillissement de la peau dû à des facteurs exogènes.

7. Utilisation cosmétique de préparations topiques selon l'une quelconque des revendications précédentes, pour le traitement d'une ou plusieurs des manifestations suivantes du vieillissement de la peau dû à des facteurs exogènes :
a) sécheresse, formation de crevasses, rugosité et formation de ridules de sécheresse, prurit et/ou regraissage réduit par les glandes sébacées (par exemple après le lavage) ;
b) élargissements visibles des vaisseaux (couperose) ;
c) flaccidité et formation de rides ;
d) hyper- et hypopigmentations et anomalies de pigmentation locales (par exemple taches de sénescence) et
e) sensibilité accrue au stress mécanique (par exemple formation de crevasses).

8. Utilisation d'associations de substances actives constituées
i) d'un composé ou de plusieurs composés choisi(s) dans le groupe des ubiquinones et
ii) de créatine.
pour la production de préparations thérapeutiques topiques destinées au traitement de la xérose sénile et/ou du vieillissement de la peau dû à des facteurs exogènes.

9. Utilisation d'associations de substances actives constituées
i) d'un composé ou de plusieurs composés choisi(s) dans le groupe des ubiquinones et
ii) de créatine.
pour la production de préparations thérapeutiques topiques destinées au traitement d'une ou plusieurs des manifestations suivantes du vieillissement de la peau dû à des facteurs exogènes :
a) sécheresse, formation de crevasses, rugosité et formation de ridules de sécheresse, prurit et/ou regraissage réduit par les glandes sébacées (par exemple après le lavage) ;
b) élargissements visibles des vaisseaux (couperose) ;
c) flaccidité et formation de rides ;
d) hyper- et hypopigmentations et anomalies de pigmentation locales (par exemple taches de sénescence) et
e) sensibilité accrue au stress mécanique (par exemple formation de crevasses).
